(19) Europäisches Patentamt
European Patent Office
Office européen des brevets

(11) Publication number: **0 148 148**
**A2**

(12) # EUROPEAN PATENT APPLICATION

(21) Application number: 84870185.0

(22) Date of filing: 20.12.84

(51) Int. Cl.[4]: C 07 C 143/46
C 07 C 139/00

(30) Priority: 27.12.83 US 566122

(43) Date of publication of application:
10.07.85 Bulletin 85/28

(84) Designated Contracting States:
BE CH DE FR GB IT LI NL SE

(71) Applicant: Monsanto Company
Patent Department 800 North Lindbergh Boulevard
St. Louis Missouri 63167(US)

(72) Inventor: Lynch, Gary James
2715 Bellecote
St. Louis Missouri 63114(US)

(72) Inventor: Shen, Chung Yu
12630 Conway Downs
St. Louis Missouri 63141(US)

(74) Representative: Lunt, John Cooper et al,
Monsanto Europe S.A. Patent Department Avenue de Tervuren 270-272 Letter Box No 1
B-1150 Brussels(BE)

(54) **Production of sodium alkanoyloxybenzene sulfonate.**

(57) A process is provided for preparing sodium alkanoyloxybenzene sulfonate by the reaction of anhydrous sodium phenol sulfonate with an alkanoylhalide in the substantial absence of solvent or inert reaction medium. The sodium alkanoyloxybenzene sulfonates are useful in detergents.

EP 0 148 148 A2

Croydon Printing Company Ltd

# PRODUCTION OF SODIUM ALKANOYLOXYBENZENE SULFONATE

## BACKGROUND OF THE INVENTION

This invention relates to the production of sodium alkanoyloxybenzene sulfonate. More particularly, it relates to a process for preparing sodium alkanoyloxybenzene sulfonate by the reaction of sodium phenol sulfonate with alkanoylhalide. Sodium alkanoyloxybenzene sulfonates are useful components in detergent compositions which requires that the materials have good assay, acceptable color and are free of offensive odors.

U.S. Patent 3,503,888 issued March 31, 1970 to R. O. Miller et al discloses the use of sodium alkanoyloxybenzene sulfonates as the major component in a toilet bar. This patent discloses the preparation of sodium alkanoyloxybenzene sulfonate by reacting a fatty acid chloride with a phenol sulfonic acid in an organic solvent. The reaction mixture is then sparged with nitrogen until all HCl of the reaction mixture is removed and the reaction mixture is then neutralized to a pH of about 6.5 to 8.5 step wise with cooling and stirring to prevent overheating. When the sulfonic acid is neutralized, some of the ester is saponified and there is then produced a mixture of salts of the sulfonated phenolic ester, the sulfonated phenol and the fatty acid containing some free fatty acid.

Sodium undecanoyloxybenzene sulfonate has been prepared by the reaction of undecanoyl chloride with sodium phenol sulfonate in the presence of organic solvents such as dimethyl formamide. (F. Puschel and O. Todorov, Tenside 7 (1970) 249-54, 252.)

The foregoing methods of producing sodium alkanoyloxybenzene sulfonates employing an organic solvent reaction medium suffer the disadvantages of

requiring expensive organic solvents, long reaction time and extensive separation and cleanup procedures to obtain the required product and to recover the solvent. The use of low levels of some organic solvents as reaction medium often results in the formation of gelled reaction products which is difficult to separate to recover the desired solid product.

These disadvantages are overcome by the present invention. The preparation of sodium alkanoyloxybenzene sulfonate in accordance with the present invention is advantageous in that it is conducted without the use of solvents as the reaction medium. Because one of the reactants is a solid and the final product is a free-flowing solid it is surprising that near quantitative conversion and yield can be accomplished in a very short time. Further, the desired product is obtained and recovered in a form suitable for use in detergent products without employing tedious separation and purification procedures.

## SUMMARY OF THE INVENTION

These and other advantages are achieved by a process for preparing sodium alkánoyloxybenzene sulfonates comprising reacting substantially solid anhydrous sodium phenol sulfonate with alkanoylhalide at a temperature in the range of about 90°C to about 200°C in the substantial absence of a solvent or inert reaction medium.

The alkanoylhalide can be the chloride or bromide, the chloride is preferred. This reaction is shown schematically as follows:

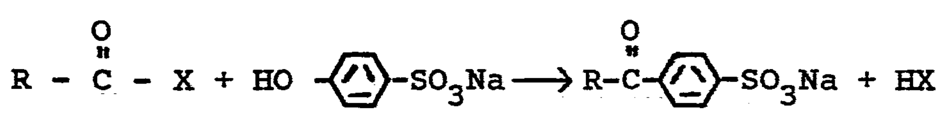

wherein R represents the aliphatic moiety of a fatty acid and mixtures thereof and X is chloro or bromo.

R can be straight or branched-chain. Suitable straight chain fatty acids are $C_6$ - $C_{12}$ fatty acids, such as hexanoic, heptanoic, octanoic, nonanoic, decanoic, hendecanoic and dodecanoic, as well as mixtures thereof. Particularly suitable mixtures are the $C_8$-$C_{10}$ mixtures which can be derived from coconut oil. Suitable branched-chain fatty acids are $C_6$ -$C_{12}$ iso fatty acids having up to 3 branches, for example, 2 ethyl-hexanoic and branched acids derived from branched olefins after hydrocarbonylation and oxidation or hydrocarboxylation and the like. Preferably the straight or branched chain fatty acids have from 7 to 10 carbon atoms. Particularly preferred are the $C_8$ to $C_{10}$ fatty acids and mixtures thereof.

DETAILED DESCRIPTION OF THE INVENTION

It has been found that sodium alkanoyloxybenzene sulfonates and particularly the $C_6$ - $C_{12}$ alkanoyloxybenzene sulfonates can be efficiently prepared in high yields and high purity in the substantial absence of solvent or inert reaction medium by reacting anhydrous sodium phenol sulfonate, mainly para and ortho isomers, with alkanoyl halide at a temperature in the range of about 90°C to about 200°C. The process in accordance with this invention can be conducted as a slurry reaction or conducted as a semi-dry solid reaction.

The process can be conducted using stoichiometric amounts of the reactants or a stoichiometric excess of either of the reactants. It is preferred to employ an excess of the alkanoylhalide reactant to obtain substantially complete conversion of the particulate solid sodium phenol sulfonate. The excess alkanoylhalide reactant is recovered from the reaction mass by convenient methods such as filtration, centrifugation, solvent extraction, vacuum

vaporization and the like to provide substantially pure sodium alkanoyloxybenzene sulfonate.

The by-product hydrogen halide vapor is removed during the reaction and can be recovered as commercial grade acid. A dry nitrogen gas purge can be used to facilitate stripping the hydrogen halide vapor.

It is important to conduct the reaction under substantially anhydrous conditions to minimize consumption of the alkanoylhalide to form free fatty acid by hydrolysis. The presence of significant amounts of free fatty acid in the reaction product is an undesirable contaminant because of its tendency to become rancid and produce disagreeable odors in consumer products formulated with the sodium alkanoyloxybenzene sulfonate product.

In the slurry reaction embodiment of the present invention, the reaction is conducted with excess alkanoylhalide, for example, 2 to 10 times the stoichiometric amount of the alkanoylhalide. In this manner, the reaction mass remains a slurry. The use of excess liquid alkanoylhalide reactant forms a readily flowable slurry reaction mixture with the particulate solid anhydrous sodium phenol sulfonate reactant. Upon termination of the reaction, the excess alkanoylhalide can be conveniently separated from the particulate solid sodium alkanoyloxybenzene sulfonate product and recycled in the process.

In the semi-dry or solid reaction embodiment of the present invention, the reaction is conducted with substantially stoichiometric amounts of the reactants or preferably with a slight excess of the alkanoylhalide reactant, for example 5 to 20% excess, and the reaction is characterized as a semi-dry or particulate solid reaction wherein the solid sodium phenol sulfonate is dispersed or blended with the

liquid alkanoylhalide. When stoichiometric amounts of the reactants are employed product conversion can be reduced due to the presence of residual moisture. However, use of a slight excess of the alkanoylhalide counteracts any losses due to the presence of moisture in the reactor. The reaction mixture generally is characterized initially as a slurry wherein the liquid alkanoylhalide phase is about 40 to 60% by volume, then proceeds to a viscous stage wherein the liquid stage is about 12 to about 25% by volume due to the formation of the solid product and then to a powdery stage wherein the liquid phase is less than about 12% by volume and the reaction mass resembles a free flowing powder. The high power required to agitate the reaction mixture during the viscous stage can be avoided by proportioning the total charge of the reactants into several relative proportions and charging one proportion to the stirred reactor and upon termination of the reaction at the powder stage adding serially the other proportions in such a manner to minimize the liquid volume of the reaction mixture. Alternatively, the reaction can be carried out by recycling a quantity of the finished reaction product as the initial charge to the stirred reactor and adding the reactants thereto in such proportions that the liquid volume of the reaction mixture is maintained below about 12% volume.

It is important to maintain the temperature of the reaction mixture in the range of about 90°C to about 200°C. At temperatures substantially below this range, the reaction rate is undesirably slow. At temperatures above this range, the sodium alkanoyloxybenzene sulfonate is subject to degradation and/or discoloration and undesired side reactions may occur. Preferably the reaction is conducted at temperatures in the range of about 120° to about 160°C.

Pressures below or above atmospheric pressure can be used in the process of this invention. Reduced pressures as low as about 200 torr (26.7 kPa) can be employed. At reduced pressures the removal and recovery of by-product hydrogen halide is enhanced. Increased pressures as high as about 4 atmospheres, 3040 torr (405.2kPa) can be employed. Increased pressures aid in maintaining substantially anhydrous conditions. Conducting the reaction under ambient pressures and employing a dry nitrogen purge to facilitate the removal of the hydrogen halide by-product is generally preferred.

Various conventional reactors equipped with means of heating and agitating the reaction mixture can be employed in the process of this invention. In view of the corrosive properties of the by-product hydrogen halide, the reactor should be constructed of or lined with various materials resistant to hydrogen halide vapors and fitted with means for collection and recovery of the hydrogen halide by-product. Such materials can be, for example, stainless steel, monel, high-nickel alloys, glass and the like.

As described above, the process of this invention is conducted substantially in the absence of solvent or inert medium. In this manner the use of additional materials are avoided and tedious separation and cleanup procedures are not required. For example, using 135 parts by weight of dichloroethane as the reaction medium with 25 parts by weight anhydrous sodium phenol sulfonate and 27 parts by weight nonanoyl chloride a gelled reaction mass is formed which is not readily separated to provide substantially pure product.

This invention is further illustrated by, but not limited to, the following examples wherein all

percentages and parts are by weight unless otherwise indicated.

EXAMPLE I

To a 500 ml Morton 3 neck flask fitted with a mechanical stirrer, nitrogen inlet stopcock adaptor and a reflux condensor protected by a foaming bulb and leading to a trap and caustic scrubber, was charged 19.6 grams of anhydrous sodium phenol sulfonate (0.1 mole) and 80.4 grams of octanoyl chloride (0.494 mole). The reaction flask was heated on an oil bath. With stirring, the reaction mixture was maintained at 120°C for about 1 hour and 15 minutes. A dry nitrogen purge was used initially and was stopped upon considerable HCl gas evolution from the reaction mixture. After about 10 minutes the gas evolution subsided and the nitrogen purge was used again. The reaction mixture was cream-like and maintained good fluidity throughout the reaction period.

The reaction mixture was allowed to cool to about 70°C and was filtered through a sintered glass funnel. The particulate solid reaction product was washed with three 50 ml portions of acetone and then dried in a vacuum oven at 120°C and 4 mm Hg(533 Pa) for about six hours to yield 29.7 grams (92.3% theory) of dry white product. The product was analyzed and contained about 98.4% by weight octanoyloxybenzenesulfonate, about 1.1% by weight octanoic acid and about 1.3% by weight sodium phenol sulfonate.

The following examples demonstrate the preparation of sodium alkanoyloxybenzene sulfonates using the semi-dry or solid reaction process of the present invention.

EXAMPLE II

This Example demonstrates the process of charging the reactants stepwise in increments.

Into a 1 liter Ace reactor fitted with a horseshoe stirrer equipped with the torque meter and indirectly heated by a silicone oil bath was charged 98.1 grams anhydrous sodium p-phenol sulfonate (0.5 mole) and 101.6 grams of nonanoyl chloride (0.576 mole, 15% excess). The reaction mixture was stirred and heated to about 120°C indirectly with the oil bath at about 130°C. Within about 4 minutes the reaction mixture foamed to about 1/3 the reactor volume. After about 6 minutes the reaction mass changed into a dry powder. The reaction was continued at 120°C for about 10 minutes and a sample of about 1.6 grams was drawn for analysis.

Step 2. Another 98.1 grams of anhydrous sodium p-phenol sulfonate and 101.6 grams of nonanoyl chloride were charged to the above reaction mass. The reaction mass changed to a slightly sticky mass for about 1 to 2 minutes before changing to a powder. The torque meter scale was a maximum of 180 and it was noted that the torque increased from about 30 to 60 during agitation of the sticky mass. The reaction was continued for about 10 minutes and a sample of about 2.5 grams was taken for analysis.

Step 3. Another 98.1 gram of the phenol sulfonate and 101.6 grams of nonanoyl chloride were added to the powder reaction mass of step 2. A similar transition through the sticky mass state was observed. After about 10 minutes a sample of about 2.2 grams was taken for analysis.

Step 4. Another 98.1 grams of the phenol sulfonate and 101.6 grams of nonanoyl chloride were added. The reaction mass was more than 1/2 of the reactor volume. The torque increased from the range of 40 - 50 to 70 - 80 before dropping back to about 50. After 10 minutes a sample of about 1.9 grams was taken for analysis and the reaction mass was recovered

from the reactor. Yield as percent of theory based upon conversion of the sodium phenol sulfonate was 93.7%.

The results of the analysis of the samples follows:

| Component,wt.% | Step 1 | Step 2 | Step 3 | Step 4 |
|---|---|---|---|---|
| Nonanoic acid | 9.04 | 8.76 | 8.31 | 8.07 |
| Nonanoic chloride | 0.67 | 0.57 | 0.64 | 0.66 |
| NaPS* | 4.66 | 3.89 | 3.42 | 3.38 |
| NOBS** | 85.63 | 86.78 | 87.62 | 87.89 |

* sodium p-phenol sulfonate.

** sodium nonanoyloxybenzenesulfonate.

A sample of the product was dried in a vacuum oven at 120°C and 0.5 mm Hg(66.66Pa) pressure overnight. Analyses of the vacuum dried material showed 0.84% by weight nonanoic acid; 0.47% by weight nonanoyl chloride; 3.88% by weight sodium phenol sulfonate and 94.81% by weight sodium nonanoyloxybenzenesulfonate. The vacuum dried material had no undesirable odor and was suitable for use in consumer products.

EXAMPLE III

Using the procedures and techniques of Example 2, sodium octanoyloxybenzenesulfonate was prepared by charging 98.1 grams anhydrous sodium p-phenol sulfonate (0.5 mole) and 97.5 grams of octanoyl chloride (0.6 mole, 20% excess) in each step of the process. Prior to the first step the reactor was dried in an oven at 120°C to remove residual moisture. During step 1 the reaction mass initially foamed as a thin slurry, then turned into a wet solid. After a reaction period of about 14.5 minutes a 1.34 grams sample was taken for analysis and step 2 was initiated with the second charge. During step 2 the reaction

mass changed from a wet solid slurry to a wet solid which was difficult to agitate. After a cumulative reaction period of about 28 minutes a 1.32 grams sample was taken for analysis and the reactor was charged for step 3. During step 3 the reaction mass changed from a wet solid to a sticky solid and the agitator was stopped briefly to break away the sticky solid. After a cumulative reaction period of about 44 minutes a 1.56 grams sample was taken for analysis and the reactor was charged for the final step 4. In step 4 the wet solid reaction mass remained sticky and is difficult to break away from the agitator. After a cumulative reaction period of about 55 minutes a 1.29 grams sample for analysis was taken and the reaction mass was recovered from the reactor. Yield and percent of theory based upon conversion of the sodium phenol sulfonate was 100%. Results of analysis of the samples follows:

| Component,wt.% | Step 1 | Step 2 | Step 3 | Step 4 |
|---|---|---|---|---|
| Octanoic acid | 5.75 | 15.21 | 3.56 | 3.19 |
| Octanoyl chloride | 3.57 | 2.73 | 3.50 | 4.94 |
| NaPS* | 4.39 | 2.55 | 0.63 | 0.00 |
| OOBS** | 86.29 | 79.51 | 92.31 | 91.87 |

* sodium p-phenol sulfonate.

** sodium octanoyloxybenzenesulfonate.

A sample of the final product, after step 4, was dried in a vacuum oven at 120°C and 0.5 mm Hg(66.66Pa) pressure overnight. Analysis of the vacuum dried product showed 0.43% by weight octanoic acid and 99.57% by weight sodium octanoyloxybenzene sulfonate.

The following Example shows an alternative semi-dry process using an initial charge of the anhydrous sodium phenol sulfonate and adding the fatty acid chloride in increments.

EXAMPLE IV

With agitation 93.4 grams of octanoyl chloride (0.575 mole, 15% excess) were added in three equal shots about 15 minutes apart to a 1 litre jacketed stainless steel sigma-blade reactor containing 115.5 grams anhydrous sodium phenol sulfonate (0.5 mole) maintained at $120^{o}$-$140^{o}$C. Dry nitrogen gas was used to purge out the by-product HCl. The reaction product was discharged after a cumulative reaction period of 45 minutes. Analysis of a sample of the semi-dry solid product showed 2.79% by weight octanoic acid; 0.39% by weight octanoyl chloride; 2.15% by weight sodium phenol sulfonate and 94.67% by weight sodium octanoyloxybenzene sulfonate.

In the foregoing Examples, the samples were analyzed by the following titration method.

a) Weigh 1 to 6 grams of the sodium alkanoyloxybenzene sulfonate and dissolve it in 100 grams of a 50/50 mixture of tetrahydrofuran and deionized water. Immediately titrate the solution with 0.2 N NaOH using an automatic titrator recording the volume of titrant and pH. An initial pH lower than about 3.8 indicates that the sample is likely to contain free fatty acid chloride. The titration curve will show 3 breaks at pH approximately 4.5, 8.0, and 11.0

b) The first break titrates free HCl from fatty acid chloride.

$$\text{Wt \% fatty acid chloride} = \frac{(CC)(N)(M.W)\ (100)}{(\text{Sample weight})\ (1000)}$$

where M.W. is the molecular weight of the fatty acid chloride.

c) The second break represents fatty acid.

$$\text{Wt \% fatty acid} = \frac{[(CCpH=8)-2(CCpH=4.5)](N)(M.W.)(100)}{(\text{Sample weight})\ (1000)}$$

where M.W. is the molecular weight of the fatty acid.

d) The final break represents the titration of the phenol group in sodium phenol sulfonate.

$$\text{Wt.\% NaPS} = \frac{[(CC\ pH=11) - (CC\ pH=8)](N)(M.W.)(100)}{(\text{Sample weight})\ (1000)}$$

where M.W. is the molecular weight of NaPS which is 196.

e) Wt. % of the sodium alkanoyloxybenzene sulfonate = 100 - (fatty acid chloride + fatty acid + NaPS).

The clean-up procedures employed to provide acceptable product produced in accordance with this invention are conventional efficient procedures. For example, the sodium alkanoyloxybenzene sulfonate can be vacuum dried at temperatures in the range of about 120°C to less than about 200°C at a pressure in the range of 0.5 mm Hg(66.6 Pa) to 100 mm Hg(13.3 kPa) for a period of about 10 minutes to about 60 minutes to remove the volatile free fatty acid and fatty acid halide. Particularly good results are achieved by vacuum drying at 150°C - 170°C at 10 mm Hg(1.33 kPa) for 15 to 20 minutes. In this manner, a product containing less than 1% combined weight of free fatty acid and fatty acid halide can be obtained. Alternatively, the product can be washed with the solvent in which the product is minimally soluble and the free fatty acid and fatty acid chloride are sufficiently soluble for extraction from the product. Suitable solvents include hexane, toluene, methylene chloride, acetone and the like. Of course, a

combination of solvent wash and vacuum drying can be used with good results.

Although the invention has been described in terms of specified embodiments which are set forth in considerable detail, it should be understood that this is by way of illustration only and that the invention is not necessarily limited thereto since alternative embodiments in operating techniques will become apparent to those skilled in the art in view of the disclosure. Accordingly, modifications are contemplated which can be made without departing from the spirit of the described invention.

WEAT IS CLAIMED IS:

1. A process for preparing sodium alkanoyloxybenzene sulfonate which comprises reacting substantially anhydrous sodium phenol sulfonate with an alkanoylhalide at a temperature in the range of about 90°C to about 200°C in the substantial absence of solvent or inert reaction medium.

2. The process of Claim 1 wherein the reaction is conducted under substantially anhydrous conditions.

3. The process of Claim 2 wherein the alkanoylhalide is $C_6$-$C_{12}$ alkanoylchloride or mixtures thereof.

4. The process of Claim 3 wherein the temperature is in the range of about 120°C to about 160°C.

5. The process of Claim 4 wherein the reaction is conducted with a stoichiometric excess of the alkanoylchloride.

6. The process of Claim 5 wherein the alkanoylchloride is octanoyl or nonanoyl chloride.

7. A slurry process for preparing sodium alkanoyloxybenzene sulfonate which comprises mixing under substantially anhydrous reaction conditions sodium para-phenol sulfonate with at least twice the stoichiometric amount of alkanoylhalide at a temperature in the range of about 90°C to about 200°C in the substantial absence of solvent or inert reaction medium.

8. The proess of Claim7 wherein the temperature is in the range of about 120°C to 160°C.

9. The process of Claim 8 wherein the alkanoylhalide is $C_6$ - $C_{12}$ alkanoylchloride or mixtures thereof.

10. The process of Claim 9 wherein the alkanoylchloride is octanoyl chloride or nonanoyl chloride.

11. A semi-dry process for producing sodium alkanoyloxybenzene sulfonate which comprises mixing under substantially anhydrous reaction conditions sodium para-phenol sulfonate with about 5% to about 20% stoichiometric excess of alkanoylhalide at a temperature in the range of about 90°C to about 200°C in the substantial absence of solvent or inert reaction medium.

12. The process of Claim 11 wherein the alkanoylhalide is $C_6$ - $C_{12}$ alkanoylchloride or mixtures thereof.

13. The process of Claim 12 wherein the temperature is in the range of about 120°C to about 160°C.

14. The process of Claim 13 wherein the alkanoylchloride is octanoyl or nonanoyl chloride.

15. The process of Claim 13 wherein the reaction is conducted stepwise by adding to the sodium para-phenol sulfonate with sufficient agitation the alkanoylchloride in two or more proportions at sufficient time intervals to maintain a substantially dry flowable reaction mass.

16. The process of Claim 15 wherein the alkanoylchloride is added in three proportions.

17. The process of Claim 15 wherein the alkanoylchloride is nonanoyl chloride.

18. The process of Claim 15 wherein the alkanoylchloride is octanoyl chloride.

19. The process of Claim 15 wherein the time intervals are in the range of about 10 minutes to about 30 minutes.

20. The process of Claim 19 wherein the alkanoylchloride is octanoyl or nonanoyl chloride.